# DEMANDE DE BREVET EUROPEEN

(11) **EP 1 738 742 A2**
(43) Date de publication de la demande: **03.01.2007**
(21) Numéro de dépôt: 06115286.4
(22) Date de dépôt: 12.06.2006
(51) Int. Cl.: A61K 8/49, A61K 31/426, A61Q 7/00, A61P 17/04

(54) **Composés benzyl-1,3-thiazolidine-2,4-diones , leurs utilisations et compositions pour stimuler ou induire la pousse des fibres kératiniques et/ou freiner leur chute**

(30) Priorité: 28.06.2005 FR 0551789
(71) Demandeur: L'Oreal, 75008 Paris (FR)
(72) Inventeur: Boulle, Christophe, Lagny S/Marne, 77400 (FR); Dalko, Maria, 91190, Gif S/Yvette (FR)
(74) Mandataire: Miszputen, Laurent

(57) **Abrégé**

L'invention a pour objet l'utilisation comme agent pour induire et/ou stimuler la croissance des fibres kératiniques notamment humaines et/ou freiner leur chute et/ou augmenter leur densité, d'une quantité efficace d'un composé benzyl-1,3-thiazolidine-2,4-dione suivante :

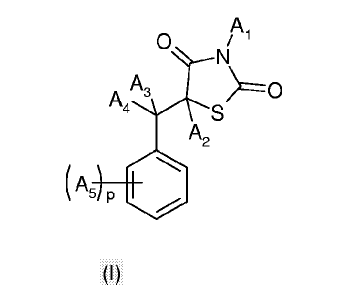

L'invention a trait également à une composition de soin ou de maquillage des cheveux ou des cils, destinée à induire et/ou stimuler leur croissance et/ou freiner leur chute.

Elle se rapporte, en outre, à de nouveaux composés benzyl-1,3-thiazolidine-2,4-diones et à un procédé de traitement cosmétique, destinés à stimuler la croissance des fibres kératiniques et en particulier des cheveux et/ou freiner leur chute.

En particulier l'invention a trait à une composition de soin et/ou de maquillage des cheveux ou des cils, à application topique, contenant un benzyl-1,3-thiazolidine-2,4-dione de formule (I), destinée à augmenter leur densité et/ou leur aspect.

## Description

### DOMAINE DE L'INVENTION

L'invention a pour objet l'utilisation comme agent pour induire et/ou stimuler la croissance des fibres kératiniques notamment humaines et/ou freiner leur chute et/ou augmenter leur densité, une quantité efficace d'un composé benzyl-1,3-thiazolidine-2,4-dione de formule particulière.

L'invention concerne également une composition de soin ou de maquillage des fibres kératiniques, contenant une quantité efficace d'un composé benzyl-1,3-thiazolidine-2,4-dione de formule particulière, destinée à augmenter la densité de ces fibres et/ou améliorer leur aspect. Plus spécialement, l'invention a trait à une composition de soin ou de maquillage des cheveux ou des cils, destinée à induire et/ou stimuler leur croissance et/ou freiner leur chute. Elle se rapporte, en outre, à de nouveaux composés benzyl-1,3-thiazolidine-2,4-diones et à un procédé de traitement cosmétique, destinés à stimuler la croissance des fibres kératiniques et en particulier des cheveux et/ou freiner leur chute.

Les fibres kératiniques humaines auxquelles s'applique l'invention sont notamment les cheveux, les sourcils, les cils, les poils de barbe, de moustache. Plus spécialement, l'invention s'applique aux cheveux et/ou aux cils humains.

En particulier l'invention a trait à une composition de soin et/ou de maquillage des cheveux ou des cils, à application topique, contenant un composé benzyl-1,3-thiazolidine-2,4-dione de formule particulière, destinée à augmenter leur densité et/ou leur aspect.

### ARRIÈRE PLAN DE L'INVENTION

Les fibres kératiniques humaines auxquelles s'applique l'invention sont notamment les cheveux, les sourcils, les cils, les poils de barbe, de moustache et les poils pubiens. Plus spécialement, l'invention s'applique aux cheveux et/ou aux cils humains.

La croissance des cheveux et leur renouvellement sont principalement déterminés par l'activité des follicules pileux et de leur environnement matriciel. Leur activité est cyclique et comporte essentiellement trois phases à savoir la phase anagène, la phase catagène et la phase télogène.

A la phase anagène (phase active ou de croissance), qui dure plusieurs années et au cours de laquelle les cheveux s'allongent, succède une phase catagène très courte et transitoire qui dure quelques semaines. Au cours de cette phase, le cheveu subit une évolution, le follicule s'atrophie et son implantation dermique apparaît de plus en plus haute.

La phase terminale ou phase télogène, qui dure quelques mois, correspond à une phase de repos du follicule et le cheveu finit par tomber. A la fin de cette période de repos, un nouveau follicule est régénéré, sur place, et un autre cycle recommence.

La chevelure se renouvelle donc en permanence et sur les 150 000 cheveux environ que comporte une chevelure, 10% environ sont au repos et seront remplacés en quelques mois.

La chute ou perte naturelle des cheveux peut être estimée, en moyenne, à quelques cent cheveux par jour pour un état physiologique normal. Ce processus de renouvellement physique permanent subit une évolution naturelle au cours du vieillissement, les cheveux deviennent plus fins et leurs cycles plus courts.

En outre, différentes causes peuvent entraîner une perte importante, temporaire ou définitive, des cheveux. Il peut s'agir de chute et d'altération des cheveux au décours d'une grossesse (post partum), au cours d'états de dénutrition ou de déséquilibres alimentaires, lors d'un stress physiologique ou encore au cours d'états d'asthénie ou de dysfonctionnement hormonal comme cela peut être le cas au cours ou au décours de la ménopause. Il peut également s'agir de chute ou d'altérations des cheveux en relation avec des phénomènes saisonniers.

Il peut s'agir également d'une alopécie, qui est essentiellement due à une perturbation du renouvellement capillaire entraînant dans un premier temps l'accélération de la fréquence des cycles au détriment de la qualité des cheveux, puis de leur quantité. Les cycles de croissance successifs aboutissent à des cheveux de plus en plus fins et de plus en plus courts, se transformant peu à peu en un duvet non pigmenté. Des zones sont touchées préférentiellement, notamment les golfes temporaux ou frontaux chez l'homme, et chez les femmes, on constate une alopécie diffuse du vertex.

Le terme alopécie recouvre aussi toute une famille d'atteintes du follicule pileux ayant pour conséquence finale la perte définitive, partielle ou générale des cheveux. Il s'agit plus particulièrement de l'alopécie androgénique. Dans un nombre important de cas, la chute précoce des cheveux survient chez des sujets prédisposés génétiquement, il s'agit alors d'alopécie andro-chrono-génétique ; cette forme d'alopécie concerne notamment les hommes.

Dans certaines dermatoses du cuir chevelu à caractéristique inflammatoire, telles que par exemple le psoriasis ou les dermatites séborrhéïques, la chute des cheveux peut être fortement accentuée ou entraîner des cycles des follicules fortement perturbés.

On recherche depuis de nombreuses années, dans l'industrie cosmétique ou pharmaceutique, des compositions permettant de supprimer ou de réduire l'alopécie, et notamment d'induire ou de stimuler la croissance des cheveux ou de diminuer leur chute.

Dans cette optique, on a déjà proposé un grand nombre de compositions comprenant des actifs très divers, comme par exemple le 2,4-diamino 6-piperidinopyrimidine 3-oxyde ou "minoxidil" décrit dans les brevets US 4 139 619 et US 4 596 812 ou encore ses nombreux dérivés comme ceux décrits dans les documents EP 0353123, EP 0356271, EP 0408442, EP 0522964, EP 0420707, EP 0459890, EP 0519819.

Des études cliniques ont démontré que des analogues de PGF2-α avaient la propriété de provoquer la croissance de poils et de cils chez l'homme et chez l'animal (Murray A. and Johnstone M.D., 1997. Am. J. Opht., 124(4), 544-547). Chez l'homme, des essais réalisés sur le cuir chevelu ont montré qu'un analogue de prostaglandine E2 (le viprostol) avait la propriété d'augmenter la densité capillaire (Roenigk H.H., 1988. Clinic Dermatol., 6(4), 119-121)

Par ailleurs, le document WO 98/33497 décrit des compositions pharmaceutiques contenant des prostaglandines ou des dérivés de prostaglandines, destinées à lutter contre la chute des cheveux chez l'homme. Les prostaglandines du type A2, F2α et E2 sont mentionnées comme préférées.

Cependant, les prostaglandines sont des molécules au temps de demi-vie biologique très court et agissant de façon autocrine ou paracrine, ceci traduisant le caractère local et labile du métabolisme des prostaglandines (Narumiya S. et al., 1999, Physiol. Rev., 79(4), 1193-1226).

Il apparaît donc comme important, pour maintenir et/ou augmenter la densité capillaire chez l'homme, de préserver les réserves endogènes de PGF2-α comme de PGE2 des différents compartiments du follicule pileux ou de son environnement cutané proche.

Une solution donnant de bons résultats est l'administration de composés inhibiteurs de lipoxygénase et/ou inducteurs de la cyclo-oxygénase en vue de favoriser la croissance des cheveux ; une hypothèse est que l'administration de tels composés oriente le métabolisme des acides gras vers la synthèse endogène de prostaglandines de préférence à d'autres voies.

Toutefois, pour améliorer encore les résultats, il serait souhaitable de pouvoir prolonger l'activité des prostaglandines impliquées dans la croissance et le maintien du cheveu en vie.

Il est par ailleurs bien connu que les programmes de différenciation des kératinocytes de l'épiderme et du follicule pileux sont clairement différents. Ainsi, il est connu que les kératines de la tige pilaire représente une famille (Langbein et al., 2001, J. Biol. Chem. 276 : 35123-35132) distincte de celle exprimée dans l'épiderme, que les marqueurs de différenciation tels que les kératines K₁ et K₁₀ ne sont pas exprimés dans le follicule pileux et en particulier dans la gaine externe (Lenoir et al., 1988, Dev. Biol. 130 : 610-620), que la trichohyaline (O'Guin et al., 1992, J. Invest. Dermatol. 98 : 24-32) et la kératine K6irs (Porter et al., 2001, Br. J. Dermatol. 145 : 558-568) sont exprimées dans le follicule pileux en particulier dans la gaine interne mais pas dans l'épiderme, et que la cyclo-oxygénase de type 1, si elle est exprimée dans l'épiderme, ne l'est pas dans les kératinocytes du follicule pileux mais dans la papille dermique (Michelet. et al., 1997, J. Invest. Dermatol. 108 : 205-209).

Le demandeur a maintenant mis en évidence qu'une enzyme spécifiquement impliquée dans la dégradation de ces prostaglandines est présente dans la papille dermique du cheveu, qui est un compartiment déterminant pour la vie du cheveu. En effet, le demandeur a maintenant prouvé la présence de 15-hydroxy prostaglandine déshydrogénase (15-PGDH en abréviation) de type 1 à ce niveau. Il a en outre montré que l'inhibition de la 15-PGDH de type 1 a un effet bénéfique sur la croissance pilaire.

C'est pourquoi la présente invention se rapporte à une composition de soin ou de traitement capillaire contenant au moins un inhibiteur particulier de la 15-hydroxy prostaglandine déshydrogénase de type 1 et un milieu physiologiquement acceptable.

La 15-PGDH de type 1 est une enzyme clé dans la désactivation des prostaglandines, en particulier de la PGF2-α, et de la PGE2, qui sont des médiateurs importants de la croissance et la survie du cheveu. Elle répond à la classification EC 1.1.1.141 et est NAD+ dépendante. Elle a été isolée de rein de porc ; on a notamment observé son inhibition par une hormone thyroïdienne, la tri-iodo thyronine, à des doses très supérieures aux doses physiologiques. La 15-PGDH de type 2 est quant à elle NADP dépendante.

### EXPOSÉ DE L'INVENTION

Le demandeur a trouvé que certains composés benzyl-1,3-thiazolidine-2,4-diones de formule (I) que l'on définira plus loin en détail et/ou leurs sels et/ou leurs solvates sont d'une façon surprenante dotés d'une activité favorable à l'amélioration de la densité des fibres kératiniques humaines. Il a par ailleurs trouvé que ces composés sont des inhibiteurs de la 15-hydroxy prostaglandine déshydrogénase de type 1.

La présente invention a donc pour objet l'utilisation d'une quantité efficace d'au moins un composé benzyl-1,3-thiazolidine-2,4-dione de formule (I) suivantes ou d'un de ses sels et/ou de ses solvates : 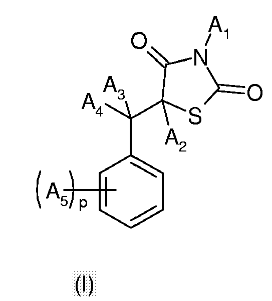
a) A₁, A₂, A₃, A₄ désignent simultanément hydrogène ;
b) A₅ désigne un groupe Z₁
c) Z₁ désigne un groupe OZ₂ dans lequel Z₂ désigne un groupe phényle éventuellement substitué par un ou plusieurs groupes adamantyle, CF₃, halogène, alkyle en C₁-C₂₀ linéaire ou ramifié, saturé ou insaturé.
d) p est compris entre 0 et 5 inclus et dans le cas où p >1, les substituants A₅ peuvent être identiques ou différents ;
   comme agent pour induire et/ou stimuler la croissance des fibres kératiniques notamment humaines et/ou freiner leur chute et/ou augmenter leur densité.

Par sels de composé de formule (I), on entend selon l'invention, les sels organiques ou inorganiques d'un composé de formule (I).

Comme sels inorganiques utilisables selon l'invention on peut citer les sels de sodium ou de potassium ainsi que les sels d'ammonium, de zinc (Zn²⁺), de calcium (Ca²⁺), de cuivre (Cu²⁺), de fer (Fe²⁺ et Fe³⁺), de strontium (Sr²⁺), de magnésium (Mg²⁺) et de manganèse (Mn²⁺) ; les hydroxydes, les halogénohydrates (chlorhydrates par exemple), les carbonates, les hydrogénocarbonates, les sulfates, les hydrogénophosphates, les phosphates.

Les sels organiques utilisables selon l'invention sont par exemple les sels de triéthanolamine, mono-éthanolamine, éthanolamine, hexadécylamine et N,N,N',N'-tétrakis-(hydroxy-propyl-2) éthylène diamine ainsi que ceux des acides organiques comme les citrates, les lactates, les glycolates, les gluconates, les acétates, les propionates, les fumarates, les oxalates et les tartrates.

Comme solvates possibles des composés de formule (I), on peut citer les hydrates, les alcoolates ou les hydroalcoolates.

Selon l'invention, les composés de formule (I) sont sous forme isolée, c'est-à-dire non polymérique.

"Au moins un" selon l'invention signifie un ou plusieurs (2, 3 ou plus). En particulier, la composition peut contenir un ou plusieurs composés de formule (I). Ce ou ces composés peuvent être des isomères cis ou trans ou Z ou E ou un mélange d'isomères cis/trans ou Z/E. Ils peuvent aussi être sous forme tautomère. Ce ou ces composés peuvent être des énantiomères et/ou des diastéréoisomères ou un mélange de ces isomères, en particulier un mélange racémique.

Par "radical alkyle" on entend au sens de l'invention un radical hydrocarboné qui peut être linéaire ou ramifié et saturé ou insaturé. En particulier, le radical alkyle comporte de 1 à 20 atomes de carbone, de préférence de 1 à 10. Comme exemple de radical alkyle utilisable dans l'invention, on peut citer les radicaux méthyle, éthyle, isopropyle, n-butyle, ter-butyle, n-hexyle, éthyle-2-hexyle, éthylène, propylène.

Comme atome d'halogène utilisable dans l'invention, on peut citer les atomes de chlore, de fluor ou de brome, et mieux les atomes de chlore et de fluor.

Parmi les composés de formule (I) conformes à l'invention, on peut citer plus particulièrement ceux choisis dans la liste de composés suivants :

| **Composé** | **Structure** |
|---|---|
| 1 | 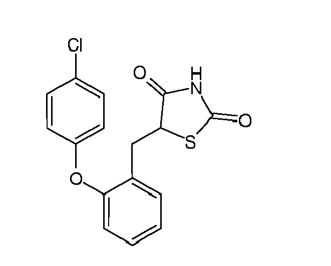 |
| 2 | 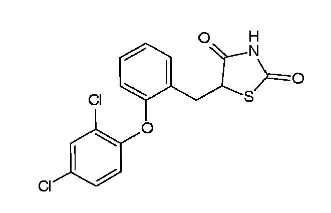 |
| 3 | 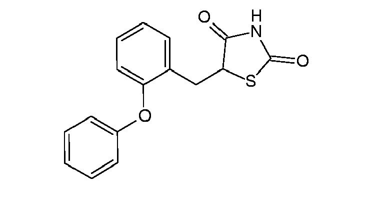 |
| 4 | 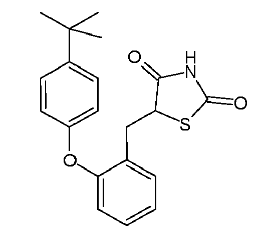 |
| 5 | 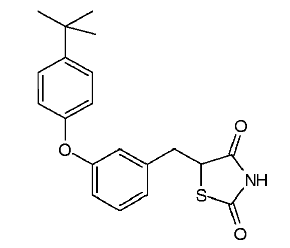 |
| 6 | 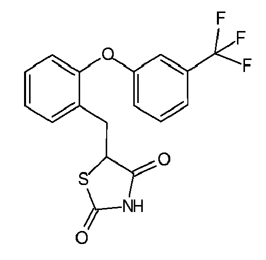 |

Les composé plus particulièrement préférés sont choisis parmi
(1) le composé 5-(2-phenoxybenzyl)-1,3-thiazolidine-2,4-dione de structure : 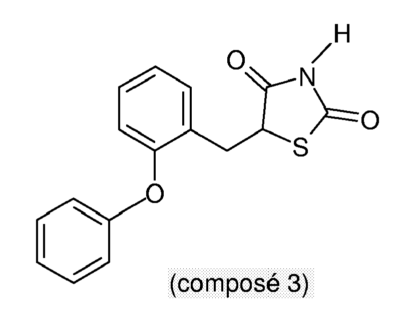
(2) le composé 5-[2-(4-tert-butylphenoxy)benzyl]-1,3-thiazolidine-2,4-dione de structure : 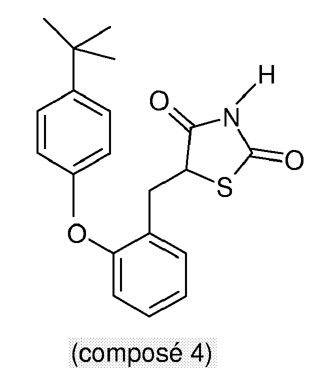
(3) le composé 5-[3-(4-tert-butylphenoxy)benzyl]-1,3-thiazolidine-2,4-dione de structure :

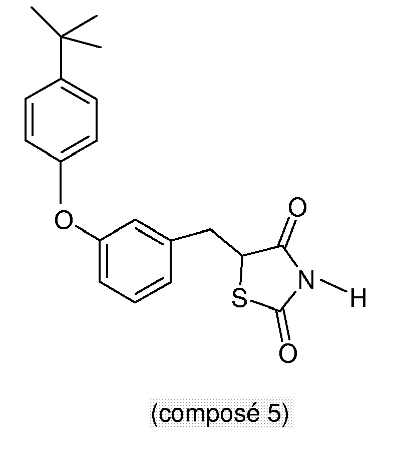

Les composés de formule (I) selon l'invention sont nouveaux et constituent un autre objet de l'invention.

Les composés de formule (I) conformes à l'invention peuvent être obtenus selon une voie de synthèse telle que décrite dans le schéma réactionnel défini ci-dessus. La voie de synthèse utilisée consiste en une substitution nucléophile aromatique, une condensation de Knoevenagel, puis une réduction de la double liaison :

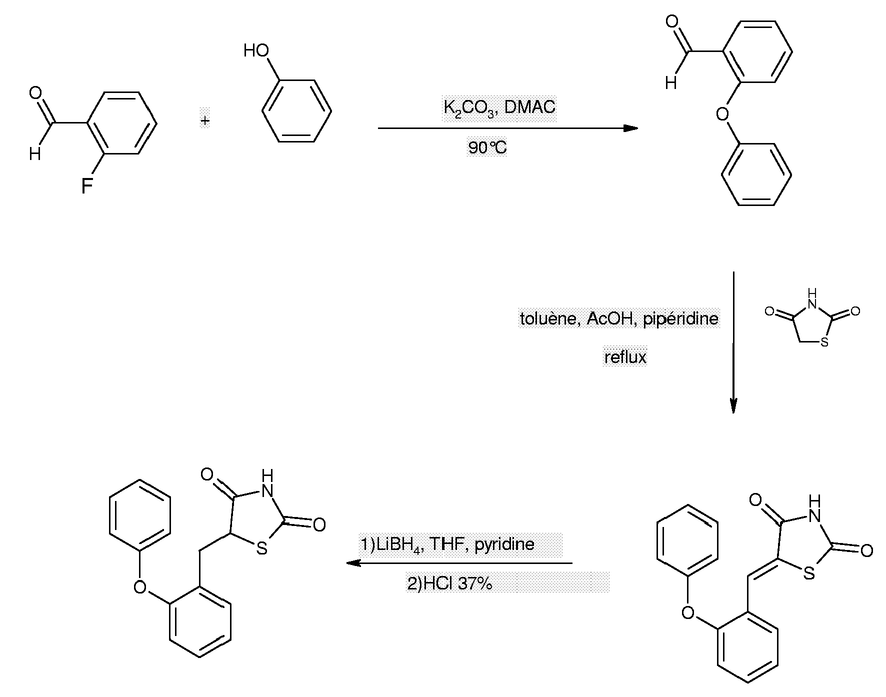

L'invention se rapporte encore à l'utilisation notamment cosmétique d'au moins un composé benzyl-1,3-thiazolidine-2,4-dione de formule (I) ou d'un de ses sels et/ou de ses solvates, tel que défini précédemment, comme agent pour induire et/ou stimuler la croissance des fibres kératiniques en particulier humaines et/ou freiner leur chute et/ou augmenter leur densité.

Elle a aussi pour objet l'utilisation cosmétique d'au moins un composé benzyl-1,3-thiazolidine-2,4-dione de formule (I) ou d'un de ses sels et/ou de ses solvates dans une composition de soin ou de traitement des fibres kératiniques notamment humaines pour réduire leur chute et/ou augmenter leur densité. Cette composition permet, en outre, de maintenir en bon état les fibres kératiniques et/ou une amélioration de leur aspect.

Elle a encore pour objet l'utilisation d'au moins un composé benzyl-1,3-thiazolidine-2,4-dione de formule (I) ou d'un de ses sels et/ou de ses solvates pour la fabrication d'une composition de soin ou de traitement des fibres kératiniques notamment humaines, destinée à induire et/ou stimuler la croissance desdites fibres et/ou freiner leur chute et/ou augmenter leur densité.

L'invention s'applique aussi aux fibres kératiniques des mammifères de l'espèce animale (chien, cheval ou chat par exemple).

Les fibres kératiniques humaines auxquelles s'applique l'invention sont notamment les cheveux, les sourcils, les cils, les poils de barbe, de moustache.

Aussi, l'invention se rapporte encore a l'utilisation cosmétique d'au moins un composé benzyl-1,3-thiazolidine-2,4-dione de formule (I) ou d'un de ses sels et/ou de ses solvates, dans une composition cosmétique de soin capillaire d'être humain pour réduire la chute des cheveux et/ou augmenter leur densité et/ou traiter l'alopécie, andro-chrono-génétique.

Elle a encore pour objet l'utilisation d'au moins un compose benzyl-1,3-thiazolidine-2,4-dione de formule (I) ou d'un de ses sels et/ou de ses solvates pour la préparation d'une composition capillaire pour être humain, destinée à induire et/ou stimuler la croissance des cheveux et/ou freiner leur chute et/ou augmenter leur densité et/ou traiter l'alopécie androgénique.

L'invention se rapporte encore à l'utilisation cosmétique d'au moins un composé benzyl-1,3-thiazolidine-2,4-dione de formule (I) ou d'un de ses sels et/ou de ses solvates, dans une composition cosmétique de soin capillaire d'être humain pour traiter l'alopécie d'origine naturelle et en particulier androgénique

L'invention se rapporte encore à l'utilisation d'au moins un composé benzyl-1,3-thiazolidine-2,4-dione de formule (I) ou d'un de ses sels et/ou de ses solvates pour la préparation d'une composition de soin capillaire d'être humain, destinée à traiter l'alopécie d'origine naturelle et en particulier androgénique. Ainsi, cette composition permet de maintenir en bon état la chevelure et/ou lutter contre la chute naturelle des cheveux et plus spécialement celle des hommes.

L'invention a encore pour objet l'utilisation cosmétique d'au moins un composé benzyl-1,3-thiazolidine-2,4-dione de formule (I) ou d'un de ses sels et/ou de ses solvates, dans une composition cosmétique de soin et/ou de maquillage des cils d'être humain, pour induire et/ou stimuler la croissance des cils et/ou augmenter leur densité

L'invention a encore pour objet l'utilisation d'au moins un composé 2 benzyl-1,3-thiazolidine-2,4-dione de formule (I) ou d'un de ses sels et/ou de ses solvates, pour la préparation d'une composition de soin ou de traitement des cils d'être humain, destinée à induire et/ou stimuler la croissance des cils et/ou augmenter leur densité. Cette composition permet ainsi de maintenir en bon état les cils et/ou améliorer leur état et/ou leur aspect.

L'invention a encore pour objet l'utilisation d'au moins un composé benzyl-1,3-thiazolidine-2,4-dione de formule (I) ou d'un de ses sels et/ou de ses solvates comme inhibiteur de la 15-hydroxy prostaglandine déshydrogénase de type 1 notamment de la peau humaine.

Elle a encore pour objet l'utilisation d'au moins un composé benzyl-1,3-thiazolidine-2,4-dione de formule (I) ou d'un de ses sels et/ou de ses solvates pour la fabrication d'une composition destinée à traiter les désordres liés à la présence de 15-hydroxy prostaglandine déshydrogénase de type 1 en particulier chez l'être humain.

La présente invention a également pour objet un procédé de traitement cosmétique des fibres kératiniques humaines (cheveux ou cils notamment) et/ou de la peau d'où émergent lesdites fibres, y compris du cuir chevelu et des paupières, caractérisé par le fait qu'il consiste à appliquer sur lesdites fibres kératiniques et/ou ladite peau, une composition cosmétique comprenant au moins un composé benzyl-1,3-thiazolidine-2,4-de formule (I) ou d'un de ses sels et/ou de ses solvates, à laisser celle-ci en contact avec les fibres kératiniques et/ou la peau d'où émergent lesdites fibres, et éventuellement à rincer lesdites fibres et/ou ladite peau.

Ce procédé de traitement présente bien les caractéristiques d'un procédé cosmétique dans la mesure où il permet d'améliorer l'esthétique des fibres kératiniques humaines et notamment des cheveux et des cils en leur donnant une plus grande vigueur et un aspect amélioré. En outre, il peut être utilisé quotidiennement pendant plusieurs mois, sans prescription médicale.

Plus spécialement, la présente invention a pour objet un procédé de soin cosmétique des cheveux et/ou du cuir chevelu humains, en vue d'améliorer leur état et/ou leur aspect, caractérisé en ce qu'il consiste à appliquer sur les cheveux et/ou le cuir chevelu, une composition cosmétique comprenant une quantité efficace d'au moins un composé benzyl-1,3-thiazolidine-2,4-dione de formule (I) ou d'un de ses sels et/ou de ses solvates, à laisser celle-ci en contact avec les cheveux et/ou le cuir chevelu, et éventuellement à rincer les cheveux et/ou le cuir chevelu.

L'invention a encore pour objet un procédé de soin cosmétique et/ou de maquillage des cils humains, en vue d'améliorer leur état et/ou leur aspect, caractérisé en ce qu'il consiste à appliquer sur les cils et/ou les paupières une composition de mascara comprenant au moins un composé benzyl-1,3-thiazolidine-2,4-dione de formule (I) ou d'un de ses sels et/ou de ses solvates et à laisser celle-ci au contact des cils et/ou les paupières. Cette composition de mascara peut être appliquée seule ou en sous-couche d'un mascara pigmenté classique et être éliminée comme un mascara pigmenté classique.

L'invention a encore pour objet une composition de soin et/ou de mascara des cheveux ou des cils contenant un milieu cosmétiquement acceptable et à application topique et un composé benzyl-1,3-thiazolidine-2,4-dione de formule (I) ou d'un de ses sels et/ou de ses solvates.

L'invention a encore pour objet une composition de soin et/ou de maquillage des fibres kératiniques, comprenant dans un milieu physiologiquement acceptable, en particulier cosmétique, au moins un composé benzyl-1,3-thiazolidine-2,4-dione ou d'un de ses sels et/ou de ses solvates et au moins un actif additionnel favorisant la repousse des fibres kératiniques humaines et/ou limitant leur chute choisi parmi l'aminexil, les agonistes du récepteur FP, les prostaglandines et leurs dérivés, et les vasodilatateurs et plus spécialement choisi parmi l'aminexil, le minoxidil, le latanoprost, l'acide (5E)-7-{(1R,2R,3R,5S)-3,5-dihydroxy-2-[(3R)-3-hydroxy-5-phényl pentyl]cyclopentyl}hept-5-énoique, le butaprost et le travoprost et également le finastéride.

L'invention a encore pour objet l'utilisation cosmétique d'un composé benzyl-1,3-thiazolidine-2,4-dione de formule (I) ou d'un de ses sels et/ou de ses solvates, comme agent pour préserver la quantité et/ou l'activité des prostaglandines au niveau du follicule pileux.

L'invention a encore pour objet l'utilisation d'au moins un composé benzyl-1,3-thiazolidine-2,4-dione de formule (I) ou d'un de ses sels et/ou de ses solvates pour la fabrication d'une composition destinée à préserver la quantité et/ou l'activité des prostaglandines au niveau du follicule pileux.

### DESCRIPTION DETAILLEE DES MODES DE REALISATION DE L'INVENTION

Par inhibiteur de la 15-hydroxy prostaglandine déshydrogénase, on entend un composé de formule (I), capable d'inhiber ou de diminuer l'activité de l'enzyme 15-PGDH de type 1, notamment humaine, et/ou capable d'inhiber, diminuer ou ralentir la réaction catalysée par cette enzyme.

Selon un mode de réalisation avantageux de l'invention, le composé de formule (I) est un inhibiteur spécifique de la 15-PGDH ; par inhibiteur spécifique on entend un actif qui est peu ou pas inhibiteur de la synthèse des prostaglandines, en particulier de la synthèse de PGF2-α ou de PGE2. Selon un mode particulier de mise en oeuvre de l'invention, l'inhibiteur de la 15-PGDH n'est peu ou pas inhibiteur de la synthèse des prostaglandines, notamment de la synthèse de PGF2-α ou de PGE2. En particulier, l'inhibiteur de 15-PGDH de type 1 n'est peu ou pas inhibiteur de la prostaglandine synthase (notée PGF synthase ou PGFS).

En effet, le demandeur a maintenant trouvé, que la PGF synthase est également exprimée dans la papille dermique. Le maintien d'une quantité efficace de prostaglandines au site d'action résulte donc d'un équilibre biologique complexe entre la synthèse et la dégradation de ces molécules. L'apport exogène de composés inhibant le catabolisme sera donc moins efficace si cette activité est combinée à une inhibition de la synthèse de ces prostaglandines.

Avantageusement, les composés de formule (I), sous forme salifiée ou non, solvatée ou non, présentent une activité inhibitrice de la 15-PGDH supérieure à l'activité d'inhibition de la PGF synthase. Ces composés sont appelés des inhibiteurs sélectifs de 15-PGDH par rapport à PGF synthase. En particulier, le rapport entre les activités inhibitrices respectivement de la PGF synthase et de la 15-PGDH pour une concentration donnée, déterminées notamment par la concentration inhibitrice de 50% de l'activité enzymatique de la PGF synthase (IC_{50fs}) par rapport à la concentration inhibitrice de 50% de l'activité enzymatique de la 15-PGDH (IC_{50dh}), est au moins supérieur à 1, et notamment d'au moins 3:1, avantageusement supérieur ou égal à 5:1.

Dans la suite du texte, et sauf mention exprès, l'emploi du terme composé de formule (I) doit être compris comme signifiant aussi bien le composé de formule (I), que l'un de ses sels, de ses solvates, en particulier hydrates ou l'un de ses sels solvatés (hydratés en particulier).

La quantité efficace d'un composé de formule (I) (salifié ou non, solvaté ou non) correspond à la quantité nécessaire pour obtenir le résultat désiré (à savoir augmenter la densité des fibres kératiniques et notamment des cheveux et des cils ou favoriser leur pousse). L'homme du métier est donc en mesure d'évaluer cette quantité efficace qui dépend de la nature du composé utilisé, de la personne à laquelle on l'applique, et du temps de cette application.

Dans la suite du texte, et sauf indication contraire, les quantités des différents ingrédients de la composition sont données en pourcentage en poids par rapport au poids total de la composition.

Pour donner un ordre de grandeur, selon l'invention, le composé de formule (I) (salifié ou non, solvaté ou non) ou un mélange de composés de formule (I) et/ou de leurs sels peut être utilisé en une quantité représentant de 10⁻³ % à 10% du poids total de la composition et préférentiellement en une quantité représentant de 10⁻³ à 5 % et mieux de 10⁻² % à 2% du poids total de la composition, par exemple de 0,5 à 2 %.

La composition de l'invention peut être à usage cosmétique ou pharmaceutique. Préférentiellement, la composition de l'invention est à usage cosmétique. Aussi, la composition doit contenir un milieu physiologiquement acceptable non toxique et susceptible d'être appliqué sur la peau, y compris le cuir chevelu et les paupières, et sur les fibres kératiniques d'êtres humains. Par "cosmétique", on entend au sens de l'invention une composition d'aspect, d'odeur et de toucher agréables.

Le composé de formule (I), salifié ou non, solvaté ou non, peut être utilisé dans une composition qui doit être ingérée, injectée ou appliquée sur la peau ou sur les fibres kératiniques (sur toute zone cutanée ou des fibres à traiter).

Selon l'invention, le composé de formule (I) ou un mélange de composés de formule (I) peut être utilisé par la voie orale en une quantité de 0,1 à 300 mg par jour, notamment de 5 à 10mg/j.

Une composition préférée de l'invention est une composition à usage cosmétique et en particulier d'application topique sur la peau et les fibres kératiniques, et plus spécialement sur le cuir chevelu, les cheveux et les cils.

Cette composition peut se présenter sous toutes formes galéniques connues adaptées au mode d'utilisation.

Pour une application topique sur la peau ou les fibres kératiniques, la composition peut avoir la forme d'une solution ou suspension aqueuse, alcoolique ou hydro-alcoolique ou d'une suspension ou solution huileuse, d'une émulsion ou dispersion de consistance plus ou moins fluide et notamment liquide ou semi-liquide, obtenue par dispersion d'une phase grasse dans une phase aqueuse (H/E) ou inversement (E/H), d'une émulsion ou dispersion solide (H/E) ou (E/H), d'un gel aqueux, hydro-alcoolique ou huileux plus ou moins fluide ou solide, d'une poudre libre ou compactée à utiliser telle quelle ou à incorporer dans un milieu physiologiquement acceptable, ou encore de microcapsules ou microparticules, de dispersions vésiculaires de type ionique et/ou non ionique.

On peut également envisager une composition sous forme de mousse ou encore sous forme de spray ou d'aérosol comprenant alors un agent propulseur sous pression.

Elle peut ainsi se présenter sous forme d'une lotion, sérum, lait, crème H/E ou E/H, gel, onguent, pommade, poudre, baume, patch, tampon imbibé, pain, mousse.

En particulier la composition à application sur le cuir chevelu ou les cheveux peut se présenter sous forme d'une lotion de soin capillaire, par exemple d'application journalière ou bi-hebdomadaire, d'un shampooing ou d'un après-shampooing capillaire, en particulier d'application bi-hebdomadaire ou hebdomadaire, d'un savon liquide ou solide de nettoyage du cuir chevelu d'application journalière, d'un produit de mise en forme de la coiffure (laque, produit pour mise en pli, gel coiffant), d'un masque traitant, d'une crème ou d'un gel moussant de nettoyage des cheveux. Elle peut encore se présenter sous forme de teinture ou de mascara capillaire à appliquer au pinceau ou au peigne.

Par ailleurs, pour une application sur les cils ou les poils, la composition à laquelle s'applique l'invention peut se présenter sous forme d'un mascara, pigmenté ou non, à appliquer à la brosse sur les cils ou encore sur les poils de barbe ou de moustache.

Pour une composition à usage par injection, la composition peut se présenter sous forme de lotion aqueuse ou de suspension huileuse. Pour un usage par voie orale, la composition peut se présenter sous forme de capsules, de granulés, de sirops buvables ou de comprimés.

Selon un mode de réalisation particulier, la composition selon l'invention se présente sous forme de crème ou lotion capillaire, de shampooing ou d'après-shampooing capillaire, de mascara capillaire ou pour cils.

Les quantités des différents constituants de la composition selon l'invention sont celles généralement utilisées dans les domaines considérés. En outre, ces compositions sont préparées selon les méthodes usuelles.

Lorsque la composition est une émulsion, la proportion de la phase grasse peut aller de 2 % à 80 % en poids, et de préférence de 5 % à 50 % en poids par rapport au poids total de la composition. La phase aqueuse est ajustée en fonction de la teneur en phase grasse et en composé(s) (I) ainsi que de celle des éventuels ingrédients additionnels, pour obtenir 100% en poids. En pratique la phase aqueuse représente de 5 % à 99,9% en poids.

La phase grasse peut contenir des composés gras ou huileux, liquides à température ambiante (25°C) et pression atmosphérique (760 mm de Hg), généralement appelés huiles. Ces huiles peuvent être compatibles ou non entre elles et former une phase grasse liquide macroscopiquement homogène ou un système bi- ou triphasique.

La phase grasse peut, en plus des huiles, contenir des cires, des gommes, des polymères lipophiles, des produits "pâteux" ou visqueux contenant des parties solides et des parties liquides.

La phase aqueuse contient de l'eau et éventuellement un ingrédient miscible en toute proportion à l'eau comme les alcools inférieurs en C₁ à C₈ tel que l'éthanol, l'isopropanol, les polyols comme le propylène glycol, le glycérol, le sorbitol ou encore l'acétone ou l'éther.

Les émulsionnants et coémulsionnants utilisés pour l'obtention d'une composition sous forme d'émulsion sont généralement utilisés dans les domaines cosmétique et pharmaceutique. Leur nature est, en outre, fonction du sens de l'émulsion. En pratique, l'émulsionnant et éventuellement le coémulsionnant sont présents, dans la composition, en une proportion allant de 0,1 % à 30 % en poids, de préférence de 0,5 à 20 % en poids et mieux de 1 à 8%. L'émulsion peut, en outre, contenir des vésicules lipidiques et notamment des liposomes.

Lorsque la composition est sous forme d'une solution ou d'un gel huileux, la phase grasse peut représenter plus de 90 % du poids total de la composition.

Avantageusement, pour une application capillaire topique, la composition est une solution ou suspension aqueuse, alcoolique ou hydro-alcoolique et mieux une solution ou suspension eau/éthanol. La fraction alcoolique peut représenter de 5% à 99,9% et mieux de 8% à 80%.

Pour une application mascara topique, la composition de l'invention est notamment sous forme d'une dispersion de cire-dans-eau ou de cire-dans-huile, d'une huile gélifiée, d'un gel aqueux, pigmenté ou non.

La composition de l'invention peut comprendre, en outre, d'autres ingrédients additionnels usuellement utilisés dans les domaines concernés, choisis parmi les solvants, les épaississants ou gélifiants de phase aqueuse ou de phase huileuse, les matières colorantes solubles dans le milieu de la composition, les particules solides du type charges ou pigments, les antioxydants, les conservateurs, les parfums, les électrolytes, les neutralisants, les polymères filmogènes, les agents bloqueurs d'U.V. comme les filtres solaires, les actifs cosmétiques et pharmaceutiques à action bénéfique pour la peau ou les fibres kératiniques, autres que les composés de formule (I), leurs mélanges. Ces additifs peuvent être présents dans la composition selon les quantités généralement utilisées dans le domaine cosmétique et dermatologique et notamment à raison de 0,01 à 50% du poids total de la composition et mieux de 0,1 à 20% et par exemple de 0,1 à 10 %. Ces additifs selon leur nature, peuvent être introduits dans la phase grasse, dans la phase aqueuse et/ou dans les vésicules lipidiques et notamment des liposomes.

Bien entendu l'homme du métier veillera à choisir les éventuels ingrédients additionnels et/ou leur quantité de telle manière que les propriétés avantageuses de la composition selon l'invention, à savoir l'inhibition de la 15-PGDH de type 1 et notamment l'augmentation de la densité des fibres kératiniques, ne soient pas ou substantiellement pas, altérées par l'adjonction envisagée.

Comme solvants utilisables dans l'invention, on peut citer les alcools inférieurs en C₂ à C₈ comme l'éthanol, l'isopropanol, le propylène glycol et certaines huiles cosmétiques légères comme les alcanes en C₆ à C₁₆ et également l'alcool benzylique.

Comme huiles utilisables dans l'invention, on peut citer les huiles d'origine minérale (huile de vaseline, isoparaffine hydrogénée), les huiles d'origine végétale (fraction liquide du beurre de karité, huile de tournesol, d'abricot, alcool ou acide gras), les huiles d'origine animale (perhydrosqualène), les huiles de synthèse (ester d'acide gras, huile de Purcellin), les huiles siliconées (polydiméthylsiloxane linéaire ou cyclique, phényltriméthicone) et les huiles fluorées (perfluoropolyéthers). Comme cires, on peut citer les cires siliconées, les cires d'abeille, de riz, de candellila, de carnauba ou paraffine, de polyéthylène.

Comme émulsionnants utilisables dans l'invention, on peut citer par exemple le stéarate ou laurate de glycérol, les stéarates ou oléates de sorbitol, les alkyl diméthiconecopolyol (avec alkyle ≥ 8) et leurs mélanges pour une émulsion E/H. On peut aussi utiliser le monostéarate ou monolaurate de polyéthylène glycol, le stéarate ou oléate de sorbitol polyoxyéthyléné, les diméthiconecopolyols et leurs mélanges pour une émulsion H/E.

Comme gélifiants hydrophiles utilisables dans l'invention, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les Bentones, les sels métalliques d'acides gras comme les stéarates d'aluminium, la silice traitée hydrophobe, l'éthylcellulose, leurs mélanges.

Comme actif cosmétique ou pharmaceutique, autre que les composés de formule (I), utilisable dans l'invention, on peut citer les actifs hydrophiles comme les protéines ou les hydrolysats de protéine, les acides aminés, les polyols, l'urée, l'allantoïne, les sucres et les dérivés de sucre, les vitamines hydrosolubles, les extraits végétaux (ceux d'Iridacées ou de soja) et les hydroxy-acides comme les acides de fruits ou l'acide salicylique ; et les actifs lipophiles, comme le rétinol (vitamine A) et ses dérivés notamment esters (palmitate de rétinol), le tocophérol (vitamine E) et ses dérivés notamment esters (acétate de tocophérol), les acides gras essentiels, les céramides, les huiles essentielles, les dérivés de l'acide salicylique comme l'octanoyl-5 salicylique, les esters des hydroxy-acides, les phospholipides comme la lécithine, leurs mélanges.

Selon un mode particulier de réalisation de l'invention, on peut associer au composé de formule (I) ou de l'un de ses sels et/ou de ses solvates, des composés additionnels favorisant la repousse et/ou limitant la chute des fibres kératiniques (cheveux, cils). Ces composés additionnels sont notamment choisis parmi les inhibiteurs de lipoxygénase tels que décrits dans EP 648488, les inhibiteurs de bradykinine décrits notamment dans EP 845700, les prostaglandines et leurs dérivés notamment ceux décrits dans WO 98/33497, WO 95/11003, JP 97-100091, JP 96-134242, les agonistes ou antagonistes des récepteurs des prostaglandines, les analogues non prostanoïques de prostaglandines tels que décrits dans EP 1175891 et EP1175890, WO 01/74307, WO 01/74313, WO 01/74314, WO 01/74315 ou WO 01/72268, leurs mélanges.

Comme autres composés actifs additionnels favorisant la pousse des fibres kératiniques et/ou limitant leur chute (notamment les cheveux ou les cils) pouvant être présents dans la composition selon l'invention on peut citer les vasodilatateurs, les antiandrogènes, les cyclosporines et leurs analogues, les antimicrobiens et antifongiques, les anti-inflammatoires, les rétinoïdes, seuls ou en mélange.

Les vasodilatateurs utilisables sont notamment les ouvreurs des canaux potassium incluant le minoxidil ainsi que les composés décrits dans les brevets US 3 382247, 5 756092, 5 772990, 5 760043, 5 466694, 5 438058, 4 973474, la cromakalim, le nicorandil et le diaxozide, seuls ou en association.

Les anti-androgènes utilisables incluent notamment les inhibiteurs stéroïdiens ou non stéroïdiens de 5α-réductase, comme le finastéride et les composés décrits dans US 5 516779, l'acétate de cyprostérone, l'acide azélaïque, ses sels et ses dérivés et les composés décrits dans US 5 480913, le flutamide, l'oxendolone, la spironolactone, le diéthylstilbestrol et les composés décrits dans les brevets US 5 411981, 5 565467 et 4910226.

Les composés antimicrobiens ou antifongiques peuvent être choisis parmi les dérivés du sélénium, l'octopirox, le triclocarban, le triclosan, le pyrithione zinc, l'itraconazole, l'acide asiatique, l'hinokitiol, la mipirocine, les tétracyclines, notamment l'érythromycine et les composés décrits dans EP 0680745, le chlorhydrate de clinycine, le peroxyde de benzoyle ou de benzyle, la minocycline et les composés appartenant à la classe des imidazoles tels que l'éconazole, le kétoconazole ou le miconazole ou leurs sels, les esters d'acide nicotinique, dont notamment le nicotinate de tocophérol, le nicotinate de benzyle et les nicotinates d'alkyles en C₁-C₆ comme les nicotinates de méthyle ou d'hexyle.

Les anti-inflammatoires peuvent être choisis parmi les anti-inflammatoires stéroïdiens comme les glucocorticoïdes, les corticostéroïdes (par exemple : l'hydrocortisone) et les anti-inflammatoires non stéroïdiens comme l'acide glycyrrhétinique et l'α-bisabolol, la benzydamine, l'acide salicylique et les composés décrits dans EP 0770399, WO 94/06434 et FR 2 268523.

Les rétinoïdes peuvent être choisis parmi l'isotrétinoïne, l'acitrétine, le tazarotène, le rétinal et l'adapalène.

Comme autres composés actifs additionnels pour favoriser la pousse et/ou limiter la chute des fibres kératiniques comme les cheveux et les cils, utilisables en association avec le composé de formule (I), salifié ou non, solvaté ou non, on peut citer l'aminexil, le 6-0-[(9Z,12Z)-octadéca-9,12-diènoyl]hexapyranose, le chlorure de benzalkonium, le chlorure de benzéthonium, le phénol, l'oestradiol, le maléate de chlorphéniramine, les dérivés de chlorophylline, le cholestérol, la cystéine, la méthionine, le menthol, l'huile de menthe poivrée, le panthoténate de calcium, le panthénol, le résorcinol, les activateurs de la protéine kinase C, les inhibiteurs de la glycosidase, les inhibiteurs de glycosaminoglycanase, les esters d'acide pyroglutamique, les acides hexosaccharidiques ou acyl-hexosaccharique, les éthylènes aryl substitués, les amino-acides N-acylés, les flavonoïdes, les dérivés et analogues d'ascomycine, les antagonistes d'histamine, les saponines, les inhibiteurs de protéoglycanase, les agonistes et antagonistes d'estrogènes, les pseudotèrines, les cytokines et les promoteurs de facteurs de croissance, les inhibiteurs d'IL-1 ou d'IL-6, les promoteurs d'IL-10, les inhibiteurs de TNF, les benzophénones et l'hydantoïne, l'acide rétinoïque ; les vitamines comme la vitamine D, les analogues de la vitamine B12, le panthoténol ; les triterpènes comme l'acide ursolique et les composés décrits dans US 5529769, US 5468888, US 5631282 ; les agents antiprurigineux comme la thénaldine, la triméprazine ou la cyproheptadine ; les antiparasitaires, en particulier le métronidazole, le crotamiton ou les pyréthrinoïdes ; les agents antagonistes de calcium, comme la cinnarizine, le diltiazem, la nimodipine, vérapamil, l'alvérine et la nifédipine ; les hormones telles que l'estriol ou ses analogues, la thyroxine et ses sels, la progestérone ; les agonistes du récepteur FP (récepteur aux prostaglandines du type F) tels que le latanoprost, l'acide (5E)-7-{(1R,2R,3R,5S)-3,5-dihydroxy-2-[(3R)-3-hydroxy-5-phényl pentyl]cyclopentyl}hept-5-énoique, le bimatoprost, le travoprost, l'unoprostone et le butaprost ; leurs mélanges.

Avantageusement, la composition selon l'invention comprend au moins un inhibiteur de la 15-PGDH tel que défini précédemment et au moins une prostaglandine ou un dérivé de prostaglandine comme par exemple les prostaglandines de la série 2 dont notamment PGF2-α et PGE2 sous forme saline ou ester (exemple les isopropyl esters), leurs dérivés comme le 16,16 diméthyl PGE2, le 17 phényl PGE2, le 16,16 diméthyl PGF2-α, le 17 phényl PGF2-α les prostaglandines de la série 1 comme le 11 déoxy prostaglandine E1, le 1 déoxy prostaglandine E1 sous forme saline ou ester, leurs analogues notamment le latanoprost, l'acide (5E)-7-{(1 R,2R,3R,5S)-3,5-dihydroxy-2-[(3R)-3-hydroxy-5-phénylpentyl]cyclopentyl}hept-5-énoique, le viprostol, le bimatoprost, le cloprosténol le travoprost, le fluprosténol, le cloprosténol, le butaprost, l'unoprostone, le misoprostol, leurs sels ou leurs esters.

De manière préférée, la composition contient au moins un agoniste non prostanoïque des récepteurs EP2 et/ou EP4 notamment tel que décrit dans EP 1175892.

On peut également envisager que la composition comprenant au moins le composé de formule (I), salifié ou non, solvaté ou non, soit sous forme liposomée, telle que notamment décrite dans le document WO 94/22468. Ainsi, le composé encapsulé dans les liposomes peut être délivré sélectivement au niveau du follicule pileux.

La composition selon l'invention peut être appliquée sur les zones alopéciques du cuir chevelu et sur les cheveux d'un individu, et éventuellement laissée en contact plusieurs heures et éventuellement rincée.

On peut, par exemple, appliquer la composition contenant une quantité efficace d'un composé de formule (I), salifié ou non, solvaté ou non, le soir, garder celle-ci au contact toute la nuit et éventuellement effectuer un shampooing le matin. Ces applications peuvent être renouvelées quotidiennement pendant un ou plusieurs mois suivant les individus.

Avantageusement, dans le procédé selon l'invention, on applique sur les zones à soigner ou traiter du cuir chevelu entre 5 et 500 µL d'une solution ou composition telle que définie précédemment, comprenant de 0,001% à 5 % d'inhibiteur de la 15-PGDH.

On va maintenant donner à titre d'illustration des exemples de réalisation de l'invention qui ne sauraient limiter en aucune façon sa portée.

### Exemple 1

### Synthèse du composé 5-(2-phenoxybenzyl)-1,3-thiazolidine-2,4-dione de formule (la) (A₁ = A₂ = A₃ = A₄ = H ; A₅ = OZ₂ ; Z₂ est phényle)

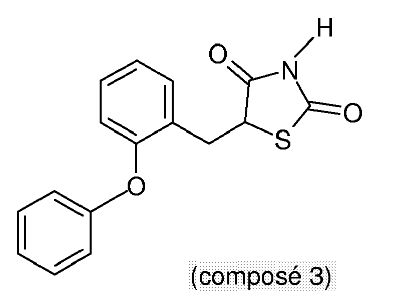

### 1 ère étape, Synthèse de 2-phénoxybenzaldéhyde :

Dans un tricol de 100mL sous argon, sont introduits le phénol (50g ; 0.531 mole), le carbonate de potassium (80.7g ; 0.584 mole) puis le diméthylacétamide (300 mL). Le 2-fluorobenzaldéhyde (55.9 mL ; 0.531 mole) est alors additionné. Le milieu réactionnel est hétérogène légèrement jaune. Ce mélange est chauffé à 120°C pendant 6 heures. Après retour à température ambiante, le milieu est dilué avec de l'eau et extrait deux fois avec du dichlorométhane. La phase organique est lavée avec une solution d'hydrogénocarbonate de sodium à 5% puis avec de l'eau et enfin avec une solution saturée en chlorure de sodium. Elle est ensuite séchée sur du sulfate de sodium puis filtrée et concentrée au maximum. L'huile jaune-marron obtenue est distillée sous vide en chauffant. 70g d'huile légèrement jaune sont obtenus (Rdt : 67%).

### 2ème étape, Synthèse de (5Z)-5-(2-phenoxybenzylidene)-1,3-thiazolidine-2,4-dione :

Dans un ballon de 100 mL surmonté d'un Dean-Stark et d'un réfrigérant, est dilué le 2-phénoxybenzaldéhyde (70g ; 0.353 mole) dans 500 mL de toluène. Sont alors ajoutés la 2,4-thiazolidinedione (41.4g ; 0.3533 mole), la pipéridine (10 mL) et l'acide acétique (10 mL). Le milieu réactionnel est porté à reflux pendant 4 heures . Après retour à température ambiante, il y a formation d'un précipité qui est filtré puis rincé plusieurs fois avec du toluène. Le produit obtenu est séché sous vide à 50 °C pour donner 100g d'un solide jaune(Rdt : 96%).

### 3ème étape, synthèse de 5-(2-phenoxybenzyl)-1,3-thiazolidine-2,4-dione :

Dans un tricol de 50mL sous argon, est dissoute la (5Z)-5-(2-phénoxybenzylidène)-1,3-thiazolidine-2,4-dione (1g ; 3.3 10-3 mole) dans le tétrahydrofurane anhydre (3 mL) et dans la pyridine (3 mL). Le borohydrure de lithium 2M (3.7 mL ; 7.4 10-3 mole) est additionné lentement, puis le milieu réactionnel est chauffé à 65°C pendant 5 heures. Après retour à 20°C, le milieu réactionnel est versé dans une solution d'acide chlorhydrique refroidie à 5°C. Le mélange est alors extrait trois fois avec de l'acétate d'éthyle. La phase organique est lavée avec une solution saturée en chlorure de sodium. Elle est ensuite séchée sur du sulfate de sodium puis filtrée et concentrée au maximum. On obtient 0,67 g d'une huile qui cristallise (Rdt : 67%). Le produit obtenu est jaune clair.

### Analyses

RMN 1H (DMSO) : 3,09 (dd, 1 H) ; 3,5 (dd, 1 H) ; 4,9 (m, 1 H) ; 6,83 (d, 1 H) ; 6,98 (dd, 2H) ; 7,12 (m, 1 H) ; 7,15 (m, 1 H) ; 7,28 (m, 1 H) ; 7,34 (dd, 1 H) ; 7,4 (m, 2H) ; 12,05 (s, 1H)

| | |
|---|---|
| Analyse élémentaire : C16H13NO3S | |
| Théorique % : | C 64,2 ; H 4,4 ; N 4,7 ; O : 16,0 ; S 10,7 |
| Mesurée % : | C 64,28 ; H 4,51 ; N 4,6 ; O : 16,19 ; S 10,61 |
| Point de fusion : | 84-85°C |

### Exemple 2

### Synthèse du composé 5-[3-(4-tert-butylphenoxy)benzyl]-1,3-thiazolidine-2,4-dione de formule (Ib) (A₁ = A₂ = A₃ = A₄ = H ; A₅ = OZ₂ ; Z₂ est phényle substitué par un groupe tert-butyle)

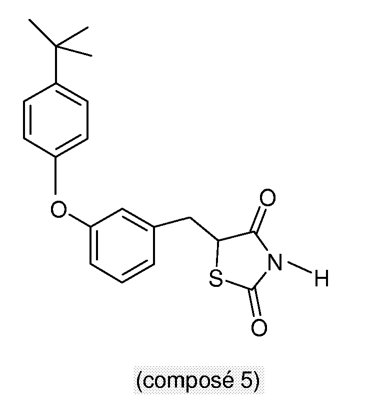

### Première étape : Synthèse de (5Z)-5-[3-(4-tert-butylphenoxy)benzylidene]-1,3-thiazolidine-2,4-dione

Dans un ballon de 250 mL surmonté d'un Dean-Stark et d'un réfrigérant, est dilué le 3-[4-(tert-butyl)phénoxy]benzaldéhyde (10g ; 39.3 10⁻³ mole) dans 120 mL de toluène puis sont additionnés la 2,4-thiazolidinedione (4.6g ; 39.9 10⁻³ mole), la pipéridine (1 mL) et l'acide acétique (1 mL). Le milieu réactionnel est porté à reflux pendant 4 heures. Après retour à température ambiante, il y a formation d'un précipité qui est filtré puis rincé plusieurs fois avec du toluène. Le solide jaune clair obtenu est séché sous vide à 50 °C pour donner 8.54g de produit (Rdt : 62%).

### Deuxième étape : Synthèse de 5-[3-(4-tert-butylphenoxy)benzyl]-1,3-thiazolidine-2,4-dione

Dans un tricol de 50mL sous argon, est dissout le (5Z)-5-[3-(4-tert-butylphénoxy)benzylidène]-1,3-thiazolidine-2,4-dione (3g ; 8.5 10-3 mole) dans le tétrahydrofurane anhydre (9 mL) et dans la pyridine (9 mL). Le borohydrure de lithium 2M (9.4 mL ; 18.7 10-3 mole) est additionné lentement, puis le milieu réactionnel chauffé à 65°C pendant 6 heures. Après retour à 20°C, le milieu réactionnel est versé lentement dans une solution d'acide chlorhydrique refroidie à 10°C. Ce mélange est extrait trois fois avec de l'acétate d'éthyle et la phase organique lavée avec une solution saturée en chlorure de sodium. Elle est ensuite séchée sur du sulfate de sodium puis filtrée et concentrée au maximum. Le produit brut est dilué dans du dichlorométhane auquel est ajouté du noir végétal. Ce mélange est agité pendant 3 heures, puis filtré sur de la célite et concentré au maximum. 1.85g d'une pâte jaune est obtenue (Rdt : 62%).

Analyses
RMN ¹H (DMSO) : 1,28 (s, 9H) ; 3,13 (dd, 1 H) ; 3,37 (dd, 1 H) ; 4,91 (dd, 1 H) ; 6,86 (dd, 1 H) ; 6,91 (m, 2H) ; 6,93 (m, 1 H) ; 7 (de, 1 H) ; 7,31 (t, 1 H) ; 7,39 (m, 2H) ; 12 (se, 1 H)

| | |
|---|---|
| Analyse élémentaire : C₂₀H₂₁NO₃S | |
| Théorique % : | C 67.6 ; H 6.0 ; N 3.9 ; O 13.5 ; S 9.0 |
| Mesurée % : | C67.31 ; H 6.03 ; N 4.04 ; O 13.69 ; S 8.79 |

### EXEMPLE 3 : Mise en évidence des propriétés inhibitrices spécifiques de la 15-PGDH des composés de formule (I)

### Test sur 15-PGDH de type 1 :

L'enzyme 15-PGDH est obtenue comme décrit dans la demande FR 02/05067 déposée au nom de L'Oréal, en suspension dans un milieu adapté à une concentration de 0,3 mg/mL puis bloquée à - 80°C. Pour les besoins du test, cette suspension est décongelée et stockée dans de la glace.

Par ailleurs on prépare un tampon Tris 100 mM, pH = 7,4, contenant 0,1 mM de dithiothréitol (D5545, Sigma-Aldrich, L'isle D'Abeau Chesne, BP 701, 38297, Saint Quentin Fallavier), 1,5 mM de β-NAD (N6522, Sigma-Aldrich, L'isle D'Abeau Chesne, BP 701, 38297, Saint Quentin Fallavier), 50 µM de Prostaglandine E₂ (P4172, Sigma-Aldrich, L'isle D'Abeau Chesne, BP 701, 38297, Saint Quentin Fallavier).

Dans la cuve d'un spectrophotomètre (Perkin-Elmer, Lambda 2) thermostaté à 37°C, dont la longueur d'onde de mesure est réglée à 340 nm, sont introduits 0,965 ml de ce tampon (préalablement porté à 37°C). 0,035 mL de suspension enzymatique à 37°C sont introduits dans la cuve concomitamment à l'enregistrement ( correspondant à une augmentation de la densité optique à 340 nm). La vitesse maximale de réaction est relevée.

Les valeurs essais (contenant les composés de formule (I)) sont comparées à la valeur témoin (sans composé de formule (I)) ; les résultats indiqués représentent soit le pourcentage d'inhibition de l'activité enzymatique de 15-PGDH pour une concentration donnée de composé de formule (I), soit la concentration à laquelle le composé de formule (I) réduit de 50% l'activité enzymatique de 15-PGDH, à savoir IC_{50dh}.

Les résultats sont donnés dans le tableau ci-après.

| **Composé** | **Structure** | **IC_{50dh} ou % inhibition à 42 µM** |
|---|---|---|
| 1 | 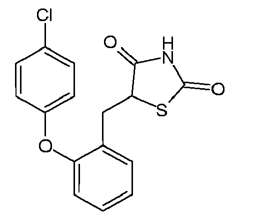 | 2,2 µM |
| 2 | 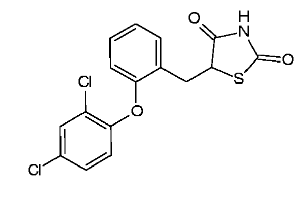 | 1,21 µM |
| 3 | 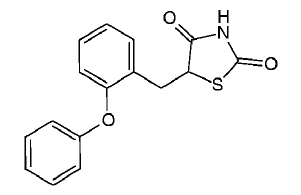 | 2,75 µM |
| 4 | 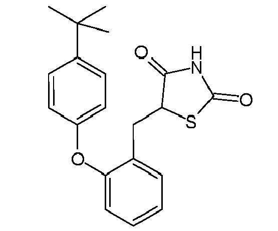 | 0,4 µM |
| 5 | 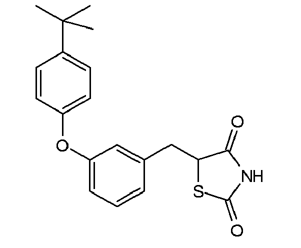 | 0,58 uM |
| 6 | 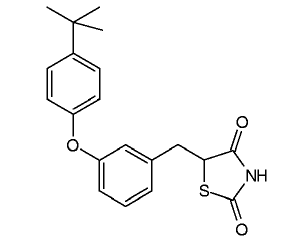 | 91% d'inhibition à 42 µM |

De ces tableaux, il ressort clairement que les composés de formule (I) sont des inhibiteurs de 15-PGDH de type 1.

Les compositions ci-après sont obtenues par les techniques habituelles couramment utilisées dans le domaine cosmétique ou pharmaceutique.

### EXEMPLE 4 : Lotion capillaire

| | | |
|---|---|---|
| 5-[2-(4-tert-butylphenoxy)benzyl]-1,3-thiazolidine-2,4-dione | | |
| (composé 4) | | 1,00 g |
| Propylène glycol | | 30,00 g |
| Alcool éthylique | | 40,00 g |
| Eau | qsp | 100,00 g |

On applique cette lotion sur le cuir chevelu, une à deux fois par jour, à raison d'1 ml par application, en massant légèrement le cuir chevelu pour faire pénétrer l'actif. La chevelure est ensuite séchée à l'air libre. Cette lotion permet de diminuer la chute des cheveux et de favoriser leur repousse. Elle permet aussi d'améliorer l'aspect des cheveux.

### EXEMPLE 5 : Lotion capillaire

| | | |
|---|---|---|
| 5-[2-(4-tert-butylphenoxy)benzyl]-1,3-thiazolidine-2,4-dione | | |
| (composé 4) | | 1,00 g |
| Alcool éthylique | | 49,00 g |
| - Eau | qsp | 100,00 g |

On applique cette lotion sur le cuir chevelu, une à deux fois par jour, à raison d'1 ml par application, en massant légèrement le cuir chevelu pour faire pénétrer l'actif. La chevelure est ensuite séchée à l'air libre. Cette lotion permet de diminuer la chute des cheveux et de favoriser leur repousse. Elle permet aussi d'améliorer l'aspect des cheveux.

### EXEMPLE 6 : Mascara cire/eau

| | | |
|---|---|---|
| Cire d'abeilles | | 6,00 % |
| Cire de paraffine | | 13,00 % |
| Huile de jojoba hydrogénée | | 2,00 % |
| Polymère filmogène hydrosoluble | | 3,00 % |
| Stéarate de triéthanolamine | | 8,00 % |
| 5-[2-(4-tert-butylphenoxy)benzyl]-1,3-thiazolidine-2,4-dione | | |
| (compose 4) | | 1,00 % |
| Pigment noir | | 5,00 % |
| Conservateur | qs | |
| Eau | qsp | 100,00 % |

Ce mascara s'applique sur les cils comme un mascara classique avec une brosse à mascara. Il permet de diminuer la chute des cils et de favoriser leur repousse. Il permet aussi d'améliorer l'aspect des cils.

### EXEMPLE 7 : Lotion capillaire

| | | |
|---|---|---|
| 5-[2-(4-tert-butylphenoxy)benzyl]-1,3-thiazolidine-2,4-dione | | |
| (compose 4) | | 0,10 g |
| Latanoprost | | 0,10 g |
| Propylène glycol | | 30,00 g |
| Alcool éthylique | | 40,00 g |
| Eau | qsp | 100,00 g |

On applique cette lotion sur le cuir chevelu, une à deux fois par jour, à raison d'1 ml par application, en massant légèrement le cuir chevelu pour faire pénétrer l'actif. La chevelure est ensuite séchée à l'air libre. Cette lotion permet de diminuer la chute des cheveux et de favoriser leur repousse. Elle permet aussi d'améliorer l'aspect des cheveux.

### EXEMPLE 8 : Lotion capillaire

| | | |
|---|---|---|
| Acide (5E)-7-{(1R,2R,3R,5S)-3,5-dihydroxy-2-[(3R)-3- | | |
| hydroxy-5-phényl pentyl]cyclopentyl}hept-5-énoique | | 0,10 g |
| 5-[2-(4-tert-butylphenoxy)benzyl] | | |
| -1,3-thiazolidine-2,4-dione | | 0,10 g |
| Propylène glycol | | 30,00 g |
| Alcool éthylique | | 40,00 g |
| Eau | qsp | 100,00 g |

On applique cette lotion sur le cuir chevelu, une à deux fois par jour, à raison d'1 ml par application, en massant légèrement le cuir chevelu pour faire pénétrer l'actif. La chevelure est ensuite séchée à l'air libre. Cette lotion permet de diminuer la chute des cheveux et de favoriser leur repousse. Elle permet aussi d'améliorer l'aspect des cheveux.

## Revendications

1. Utilisation d'une quantité efficace d'au moins un composé benzyl-1,3-thiazolidine-2,4-dione de formule (I) suivante ou d'un de ses sels et/ou de ses solvates : 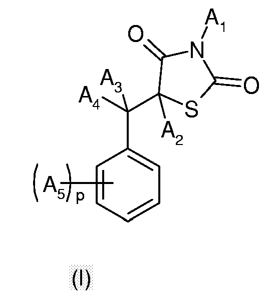 dans laquelle
a) A₁, A₂, A₃, A₄ désignent simultanément hydrogène ;
b) A₅ désigne un groupe Z₁
c) Z₁ désigne un groupe OZ₂ dans lequel Z₂ désigne un groupe phényle éventuellement substitué par un ou plusieurs groupes adamantyle, CF₃, halogène, alkyle en C₁-C₂₀ linéaire ou ramifié, saturé ou insaturé.
d) p est compris entre 0 et 5 inclus et dans le cas où p>1, les substituants A₅ peuvent être identiques ou différents ;
comme agent pour induire et/ou stimuler la croissance des fibres kératiniques notamment humaines et/ou freiner leur chute et/ou augmenter leur densité.

2. Utilisation cosmétique d'au moins un composé d'au moins un composé benzyl-1,3-thiazolidine-2,4-dione de formule (I) ou d'un de ses sels et/ou de ses solvates tel que défini dans la revendication 1 dans une composition cosmétique de soin et/ou de maquillage des fibres kératiniques notamment humaines pour réduire leur chute et/ou augmenter leur densité.

3. Utilisation d'au moins un composé d'au moins un composé benzyl-1,3-thiazolidine-2,4-dione ou de formule (I) ou d'un de ses sels et/ou de ses solvates tel que défini dans la revendication 1, pour la fabrication d'une composition de soin ou de traitement des fibres kératiniques notamment humaines, destinée à induire et/ou stimuler la croissance desdites fibres et/ou freiner leur chute et/ou augmenter leur densité.

4. Utilisation d'au moins un composé d'au moins un composé benzyl-1,3 thiazolidine-2,4-dione de formule (I) ou d'un de ses sels et/ou de ses solvates tel que défini dans la revendication 1, comme agent inhibiteur de la 15-hydroxy prostaglandine déshydrogénase de type 1, notamment humaine.

5. Utilisation d'au moins un composé d'au moins un composé benzyl-1,3-thiazolidine-2,4-dione de formule (I) ou d'un de ses sels et/ou de ses solvates tel que défini dans la revendication 1, pour la fabrication d'une composition de soin ou de traitement des fibres kératiniques notamment humaines, destinée à traiter les désordres liés à la 15-hydroxy prostaglandine déshydrogénase de type 1, notamment humaine.

6. Utilisation selon l'une des revendications précédentes, **caractérisée en ce que** les fibres kératiniques humaines sont les cheveux, les sourcils, les cils, les poils de barbe, de moustache.

7. Utilisation cosmétique d'au moins un composé benzyl-1,3-thiazolidine-2,4-dione de formule (I) ou d'un de ses sels et/ou de ses solvates tel que défini dans la revendication 1, dans une composition cosmétique de soin capillaire d'être humain pour réduire la chute des cheveux et/ou augmenter leur densité et/ou traiter l'alopécie andro-chrono-génétique et/ou traiter l'alopécie d'origine naturelle.

8. Utilisation d'au moins un composé benzyl-1,3-thiazolidine-2,4-dione de formule (I) ou d'un de ses sels et/ou de ses solvates tel que défini dans la revendication 1, pour la préparation d'une composition cosmétique capillaire d'être humain, destinée à induire et/ou stimuler la croissance des cheveux et/ou freiner leur chute et/ou augmenter leur densité et/ou traiter l'alopécie androgénique et/ou traiter l'alopécie d'origine naturelle.

9. Utilisation cosmétique d'au moins un composé benzyl-1,3-thiazolidine-2,4-dione ou de formule (I) ou d'un de ses sels et/ou de ses solvates tel que défini dans la revendication 1, dans une composition cosmétique de soin et/ou de maquillage des cils humains pour induire et/ou stimuler leur croissance et/ou augmenter leur densité.

10. Utilisation d'au moins un composé benzyl-1,3-thiazolidine-2,4-dione de formule (I) ou d'un de ses sels et/ou de ses solvates tel que défini dans la revendication 1, pour la préparation d'une composition de soin ou de traitement des cils humains, destinée à induire et/ou stimuler leur croissance et/ou augmenter leur densité.

11. Utilisation selon les revendications précédentes où les composés de formule (I) ou sont choisis parmi les composés suivants et/ou leurs sels et/ou leurs solvates :
| **Composé** | **Structure** |
|---|---|
| 1 | 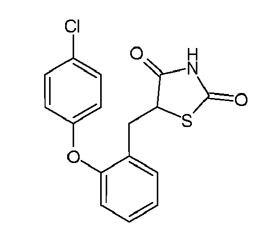 |
| 2 | 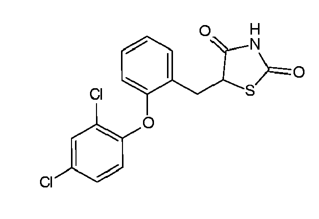 |
| 3 | 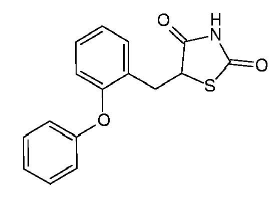 |
| 4 | 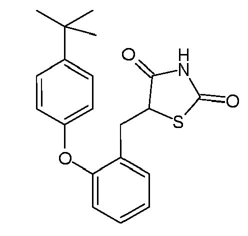 |
| 5 | 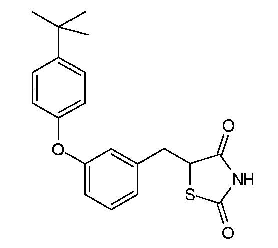 |
| 6 | 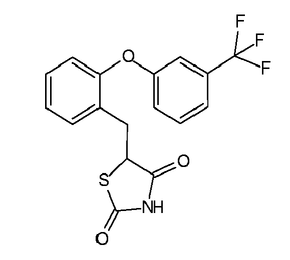 |

12. Utilisation selon les revendications précédentes où le composé de formule (I) sont est choisis parmi les composés suivants et/ou leurs sels et/ou leurs solvates :
(1) le composé 5-(2-phenoxybenzyl)-1,3-thiazolidine-2,4-dione de structure : 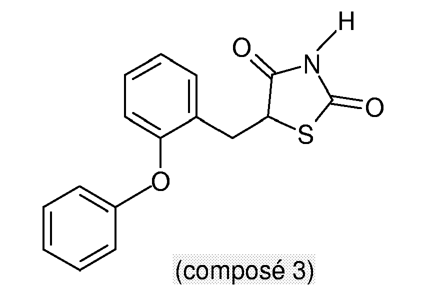
(2) le composé 5-[2-(4-tert-butylphenoxy)benzyl]-1,3-thiazolidine-2,4-dione de structure : 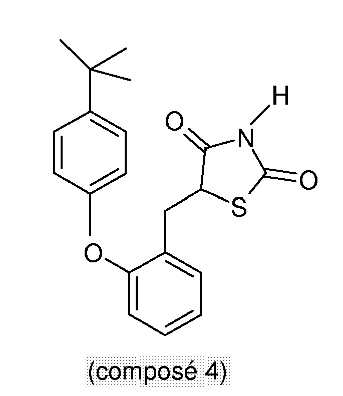
(3) le composé 5-[3-(4-tert-butylphenoxy)benzyl]-1,3-thiazolidine-2,4-dione de structure :

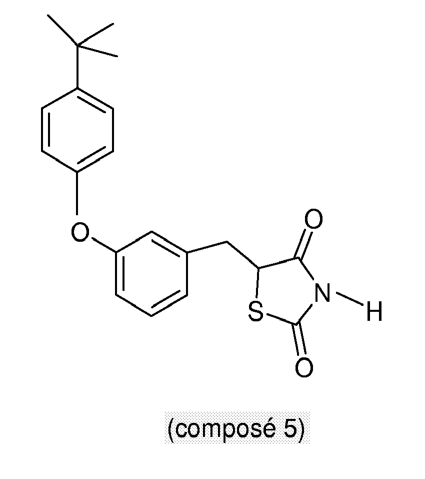

13. Utilisation selon d'une des revendications précédentes, **caractérisée en ce que** le composé de formule (I) ou un mélange de composés de formule (I) est utilisé à une concentration allant de 10⁻³ à 10 %, de préférence de 10⁻² à 2 %, par rapport au poids total de la composition.

14. Utilisation selon l'une des revendications précédentes, **caractérisée en que** la composition est une composition à application topique.

15. Composition de soin et/ou de maquillage des fibres kératiniques, contenant un milieu physiologiquement acceptable et une quantité efficace d'au moins un composé benzyl-1,3-thiazolidine-2,4-dione de formule (I) ou d'un de ses sels et/ou de ses solvates tel que défini dans l'une quelconque des revendications précédentes.

16. Composition selon la revendication 15, **caractérisée en ce que** composé de formule (I) ou un mélange de composés de formule (I) est utilisé à une concentration allant de 10⁻³ à 10 %, de préférence de 10⁻² à 2 %, par rapport au poids total de la composition.

17. Composition selon l'une des revendications 15 et 16, **caractérisée en ce qu'**elle se présente sous forme de crème ou lotion capillaire, de shampooing, d'après-shampooing, de mascara capillaire ou pour cils.

18. Composition selon l'une des revendications 15 à 17, **caractérisée en ce qu'**elle est sous forme de solution ou suspension aqueuse, alcoolique ou hydro-alcoolique.

19. Composition selon l'une des revendications 15 à 18, **caractérisée en ce qu'**elle contient d'autres ingrédients choisis parmi les solvants, les épaississants ou gélifiants de phase aqueuse ou de phase huileuse, les matières colorantes solubles dans le milieu de la composition, les charges, les pigments, les antioxydants, les conservateurs, les parfums, les électrolytes, les neutralisants, les polymères filmogènes, les agents bloqueurs d'U.V, les actifs cosmétiques et pharmaceutiques autres que les composé de formule (I), leurs mélanges.

20. Composition selon l'une des revendications 15 à 19, **caractérisée en ce qu'**elle contient, en outre, au moins un actif choisi parmi les protéines ou les hydrolysats de protéine, les acides aminés, les polyols, l'urée, l'allantoïne, les sucres et les dérivés de sucre, les vitamines hydrosolubles, les extraits végétaux, les hydroxy-acides ; le rétinol et ses dérivés, le tocophérol et ses dérivés, les acides gras essentiels, les céramides, les huiles essentielles, les dérivés de l'acide salicylique, les esters des hydroxy-acides, les phospholipides, leurs mélanges.

21. Composition selon l'une des revendications 15 à 20 **caractérisée en ce qu'**elle contient au moins un composé actif additionnel favorisant la repousse et/ou limitant la chute des fibres kératiniques.

22. Composition selon la revendication 21, **caractérisée en ce que** le composé actif additionnel est choisi parmi l'aminexil, le 6-0-(9Z,12Z)-octadéca-9,12-diènoyl]hexapyranose, les inhibiteurs de lipoxygénase, les inhibiteurs de bradykinine, les prostaglandines et leurs dérivés, les agonistes ou antagonistes des récepteurs des prostaglandines, les analogues non prostanoïques de prostaglandines, les vasodilatateurs, les antiandrogènes, le finastéride, les cyclosporines et leurs analogues, les antimicrobiens, les anti-inflammatoires, les rétinoïdes, le chlorure de benzalkonium, le chlorure de benzéthonium, le phénol, l'oestradiol, le maléate de chlorphéniramine, les dérivés de chlorophylline, le cholestérol, la cystéine, la méthionine, le menthol, l'huile de menthe poivrée, le panthoténate de calcium, le panthénol, le résorcinol, les activateurs de la protéine kinase C, les inhibiteurs de la glycosidase, les inhibiteurs de glycosaminoglycanase, les esters d'acide pyroglutamique, les acides hexosaccharidiques ou acyl-hexosaccharique, les éthylènes aryl substitués, les amino-acides N-acylés, les flavonoïdes, les dérivés et analogues d'ascomycine, les antagonistes d'histamine, les saponines, les inhibiteurs de protéoglycanase, les agonistes et antagonistes d'estrogènes, les pseudotèrines, les cytokines et les promoteurs de facteurs de croissance, les inhibiteurs d'IL-1 ou d'IL-6, les promoteurs d'IL-10, les inhibiteurs de TNF, les vitamines, les benzophénones, l'hydantoïne, l'acide rétinoïque, les agents antiprurigineux, les antiparasitaires, les antifongiques, les agents antagonistes de calcium, les hormones, les triterpènes, les agents antiandrogènes, les inhibiteurs stéroïdiens ou non stéroïdiens des 5-α-réductases, les agonistes potassiques, les agonistes du récepteur FP, leurs mélanges.

23. Composition selon la revendication 22, **caractérisée en ce que** le composé actif additionnel est choisi parmi l'aminexil, les agonistes du récepteur FP, les prostaglandines et leurs dérivés et les vasodilatateurs.

24. Composition selon l'une des revendications 23 à 24, **caractérisée en ce que** le composé actif additionnel est choisi parmi l'aminexil, le minoxidil, le latanoprost, l'acide (5E)-7-{(1R,2R,3R,5S)-3,5-dihydroxy-2-[(3R)-3-hydroxy-5-phénylpentyl]cyclopentyl}-hept-5-énoique, le butaprost et le travoprost.

25. Procédé de traitement cosmétique des fibres kératiniques humaines et/ou de la peau d'où émergent lesdites fibres, en vue d'améliorer leur état et/ou leur aspect, **caractérisé par le fait qu'**il consiste à appliquer sur les fibres kératiniques et/ou la peau, une composition cosmétique comprenant au moins un composé de formule (I) ou d'un de ses sels et/ou de ses solvates tel que défini dans l'une quelconque des revendications précédentes, à laisser celle-ci en contact avec les fibres kératiniques et/ou la peau d'où émergent lesdites fibres, et éventuellement à rincer lesdites fibres et/ou ladite peau .

26. Procédé de traitement cosmétique des cheveux et/ou du cuir chevelu, **caractérisé par le fait qu'**il consiste à appliquer sur les cheveux et/ou le cuir chevelu, une composition cosmétique comprenant une quantité efficace d'au moins un composé de formule (I) ou d'un de ses sels et/ou de ses solvates tel que défini dans l'une quelconque des revendications précédentes, à laisser celle-ci en contact avec les cheveux et/ou le cuir chevelu, et éventuellement à rincer.

27. Procédé de soin cosmétique et/ou de maquillage des cils humains, en vue d'améliorer leur état et/ou leur aspect, **caractérisé en ce qu'**il consiste à appliquer sur les cils et/ou les paupières une composition de mascara comprenant au moins un composé de formule (I) ou d'un de ses sels et/ou de ses solvates tel que défini dans l'une quelconque des revendications précédentes et à laisser celle-ci au contact des cils et/ou des paupières.

28. Composé répondant à la formule (I) suivante ou l'un de ses sels et/ou de ses solvates : 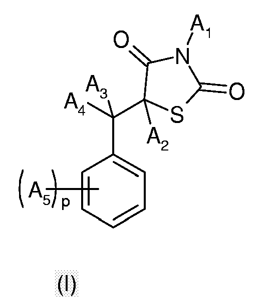 dans laquelle
a) A₁, A₂, A₃, A₄ désignent simultanément hydrogène ;
b) A₅ désigne un groupe Z₁
c) Z₁ désigne un groupe OZ₂ dans lequel Z₂ désigne un groupe phényle éventuellement substitué par un ou plusieurs groupes adamantyle, CF₃, halogène, alkyle en C₁-C₂₀ linéaire ou ramifié, saturé ou insaturé.
d) p est compris entre 0 et 5 inclus et dans le cas où p >1, les substituants A₅ peuvent être identiques ou différents.

29. Composé selon la revendication 28, choisi parmi les composés suivants :
| **Composé** | **Structure** |
|---|---|
| 1 | 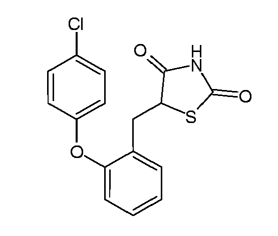 |
| 2 | 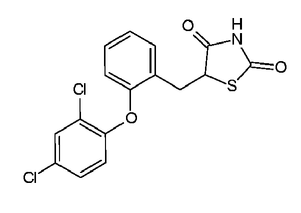 |
| 3 | 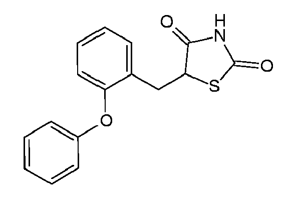 |
| 4 | 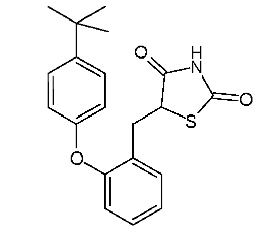 |
| 5 | 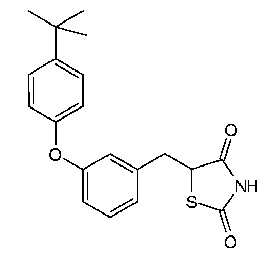 |
| 6 | 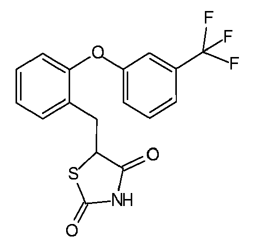 |
ainsi que leurs sels et/ou solvates.
